# NEW EUROPEAN PATENT SPECIFICATION

(11) **EP 1 339 294 B2**
(45) Date of publication and mention of the opposition decision: **21.01.2015**
(45) Mention of the grant of the patent: 23.08.2006
(21) Application number: 01996314.9
(22) Date of filing: 14.11.2001
(51) Int. Cl.: A23L 1/29, A23L 1/302, A23L 1/304, A61K 35/74, A23L 1/03, A23C 9/123, A23L 1/0528

(54) **NUTRITIONAL COMPOSITION FOR AN IMMUNE CONDITION**
NAHRUNGSZUSAMMENSETZUNG FÜR IMMUNERKRANKUNG
COMPOSITION NUTRITIONNELLE DESTINEE A UNE MALADIE IMMUNITAIRE

(30) Priority: 14.11.2000 GB 0027761
(43) Date of publication of application: 03.09.2003
(73) Proprietor: Société des Produits Nestlé S.A., 1800 Vevey (CH)
(72) Inventor: SPIVEY-KROBATH, Evelyn, 1073 Savigny (CH); CAVADINI, Claude, CH-1185 Mont-Sur-Rolle (CH); HASCHKE, Ferdinand, 82031 Grünwald (DE); JAUSSAN, Veronique, F-94300 Vincennes (FR); SCHIFFRIN, Eduardo, CH-1023 Crissier (CH)
(74) Representative: Rupp, Christian
(86) International application number: PCT/EP2001/013302
(87) International publication number: WO 2002/039834

(56) References cited:
- EP-A- 0 609 056
- EP-A- 0 704 164
- EP-A- 0 821 885
- EP-A- 0 904 784
- EP-A- 1 195 095
- WO-A-00/10402
- WO-A-00/35303
- WO-A-01/15714
- WO-A-97/34615
- WO-A-99/02170
- WO-A-99/47000
- WO-A-02/052954
- US-A- 6 093 425
- DATABASE WPI Section Ch, Week 199927 Derwent Publications Ltd., London, GB; Class B04, AN 1999-313647 XP002194844 & CN 1 208 620 A (CHEN X), 24 February 1999 (1999-02-24)
- SCHAAFSMA G ET AL: "EFFECTS OF A MILK PRODUCT, FERMENTED BY LACTOBACILLUS ACIDOPHILUS AND WITH FRUCTO-OLIGOSACCHARIDES ADDED, ON BLOOD LIPIDS IN MALE VOLUNTEERS" EUROPEAN JOURNAL OF CLINICAL NUTRITION, XX, XX, vol. 52, no. 6, June 1998 (1998-06), pages 436-440, XP002119336
- DATABASE BIOSIS [Online] BIOSCIENCES INFORMATION SERVICE, PHILADELPHIA, PA, US; SULLIVAN ASA ET AL: ""Effect of Lactobacillus paracasei on intestinal colonisation of lactobacilli, bifidobacteria and clostridium difficile in elderly persons" " XP002194824
- DATABASE MEDLINE [Online] NEMCOVA R ET AL: "Study of the effect of Lactobacillus paracasei and fructooligosaccharides on the faecal microflora in weanling piglets" XP002194825
- SHIFFRIN EJ, BRASSART D, SERVIN AL, ROCHAT F, DONNET-HUGHES A: "Immune modulation of blood leukocytes in humans by lactic acid bacteria: criteria for strain selection" AMERICAN JOURNAL OF CLINICAL NUTRITION, vol. 66, 1997, page 515S-520S XP002194826 USA
- MARTEAU P, VAERMAN JP, DEHENNIN JP, BORD S, BRASSART D, POCHART P, DESJEUX JF, RAMBAUD JC: "Effects of intrajejunal perfusion and chronic ingestion of Lactobacillus johnsonii strain La 1 on serum concentrations and jejunal secretions of immunoglobulins and serum proteins in healthy humans" GASTROENTEROL. CLIN. BIOL, vol. 21, 1997, pages 293-298, XP001062975 Paris (F)
- SPIEGEL J E ET AL: "SAFETY AND BENEFITS OF FRUCTOOLIGOSACCHARIDES AS FOOD INGREDIENTES" FOOD TECHNOLOGY, INSTITUTE OF FOOD TECHNOLOGISTS. CHICAGO, US, vol. 48, no. 1, 1994, pages 85-89, XP000423511 ISSN: 0015-6639
- SANDERS M E: "PROBIOTICS" FOOD TECHNOLOGY, INSTITUTE OF FOOD TECHNOLOGISTS. CHICAGO, US, vol. 53, no. 11, November 1999 (1999-11), pages 67-75, XP000877806 ISSN: 0015-6639

## Description

The present invention relates to a nutritional composition for prevention or treatment of an immune condition, a method of production of the composition, and use of the composition in the manufacture of a functional, food or medicament for the prevention or treatment of an immune condition in the elderly.

Within the context of this specification the word "comprises" is taken to mean "includes, among other things". It is not intended to be construed as "consists of only".

Within the context of this specification the term "an immune condition" represents an ailment selected from the group which comprises an impaired immune response, an inflammatory condition, inflammation, chronic disease (for example arthritis or gastritis), conditions associated with aging and leading to an increase of inflammatory responses.

Americans greater than 65 years old, at the turn of the century, accounted for 4% of the US population; currently, they account for greater than 12% of the population. However, although they only account for 12% of the US population, they account for greater than 40% of acute hospital bed days, buy greater than 30% of all prescription drugs and spend 30% of the US health budget. Furthermore, it has been estimated that in 2030, greater than 70 million Americans (1:5) will be over the age of 65, and those over 85 are expected to experience the highest percentage increase of all age groups.

As the average age of the population increases, obtaining a better understanding of the unique aspects of aging in relation to nutritional needs and treatment is imperative. Many physiologic functions decline progressively throughout adult life and have an impact on nutrition. For instance, a reduction in the number of functioning cells and the resultant slowing of metabolic processes results in a decrease in caloric requirements among the elderly. Also, the reduction in physical activity that generally accompanies aging further decreases energy requirements. Merely decreasing the total caloric intake of an elderly patient may adversely affect their required nutrition. When the total caloric intake is reduced, the remaining food intake must carefully insure a properly balanced intake of proteins, vitamins and minerals. To reduce caloric intake in the elderly, consumption of "empty" calories (i.e. fats) can be reduced and consumption of nutrient-dense foods (i.e. carbohydrates and proteins) can be increased.

While the nutritional needs of the mature adult differ from those of an adult, in health care settings, standard nutritional formulae are the primary form of elemental nutrition currently used. Naturally, standard formulae do not take into account the nutritional needs of an elderly patient. Standard products suffer from the problem that they must be supplemented with key micronutrients to compensate for common deficiencies and metabolic changes of an elderly patient. Therefore, a need exists for a nutritional composition which meets the nutritional needs of an elderly patient.

In addition to the above problems, a composition is required to address the problems of immune conditions in the elderly.

Furthermore, it is known that the effects of diet and nutritional supplements can play a role in improving the survival and quality of life. In particular, a need exists for a nutritional composition which can help to improve health, in particular with regard to an immune condition.

Furthermore, it is known that the problem of an impaired immune response can be associated with aging, chronic pathological conditions and/or malnutrition. This problem has been partly addressed by providing nutritional supplements. However, these supplements suffer from the problem that, generally, they are specific for certain ailments and do not provide good nutritional support for patients suffering from a more complicated combination of conditions. This is particularly relevant with respect to an elderly patient. In addition, the increase in the likelihood of chronic disease such as arthritis, gastritis, etc, with age leads to an increase in inflammatory reactions.

EP 904784 discloses a probiotic nutritional preparation for the prevention and treatment of pathogenic infections of the gastro-intestinal tract including disturbances in the immune system resulting from such infections in the general population.

Schiffrin et al (Am. J. Clin. Nutr. 1997; 66:5155-5205) shaved that the immune function of healthy adult human volunteers aged 23-62 years could be improved by supplementation of the diet with milk fermented by probiotics.

The present invention addresses the problems set out above.

Remarkably, a composition has now been found that can be used to provide nutrition to an elderly patient.

Accordingly, in a first aspect, the present invention provides a composition according to claim 1.

In a second aspect the invention provides a method of producing a composition according to an embodiment of the invention which comprises the step of blending the required nutrients together in the required amounts.

In a third aspect the invention provides the use of a composition according to an embodiment of the invention in the manufacture of a functional food or a medicament for the prevention or treatment of an immune condition in as elderly patient.

Preferably, an embodiment of a composition according to the invention comprises one or more nutrients or minerals selected from the group consisting of vitamin E, vitamin C, vitamin B6, folic acid, vitamin B12, copper, zinc and selenium. More preferably, it comprises at least two of these nutrients or minerals. Even more preferably, it comprises at least three of these nutrients or minerals. Most preferably it includes all of these minerals or nutrients.

Preferably, an embodiment of a composition according to the invention additionally comprises one or more nutrients or minerals selected from the group consisting of calcium, phosphorus, magnesium, iron, vitamin A, vitamin B 1, vitamin B2, niacin and vitamin D. More preferably, it comprises at least two of these nutrients or minerals. Even more preferably, it comprises at least three of these nutrients or minerals. Most preferably an embodiment of a composition according to the invention includes all of these minerals or nutrients.

Preferably, an embodiment of a composition according to the invention comprises calcium in the form of milk calcium or calcium derived from milk calcium.

The composition according to the invention comprises as a probiotic lactic acid bacteria ST11 (deposited under the number NCCMI-2116).

Preferably, an embodiment of a composition according to the invention comprises the following masses or amounts of nutrients or minerals (if present) per 300g of the composition. Preferably, this is the amount of composition administered per day.

Vitamin E: preferably 1 IU to 400 IU, most preferably 120 IU Vitamin C: preferably 6 mg to 300 mg, most preferably 120 mg Vitamin B6: preferably 0.17 mg to 3 mg, most preferably 2 mg Folic acid: preferably 40 ug to 800 ug, most preferably 400 ug Vitamin B12: preferably 0.24 mg to 5mg, most preferably 3.8 ug Copper: preferably 0.3 mg to 3 mg, most preferably 1.5 mg Zinc: preferably 1.5 mg to 15 mg, most preferably 15 mg Selenium: preferably 7 ug to 300 ug, most preferably 100 ug Fructo-oligosaccharides and/or gum acacia: preferably 4 g to 50 g, most preferably 6g Lactic acid bacteria: preferably 10E8 to 10E12 cfu, most preferably 10E10 cfu.

Preferably, an embodiment of a composition according to the invention comprises one or more nutrients selected from the following masses or amounts of nutrients or minerals (if present) per 300g of the composition. Preferably, this is the amount of composition administered per day.
calcium: 100mg to 300 mg, more preferably 200mg;
phosphorus: 50mg to 615mg, more preferably 150mg ;
magnesium: 110mg to 210mg, more preferably 60mg;
vitamin A: 1000IU to 1500IU, more preferably 1333IU;
vitamin D: 50IU to 150IU, more preferably 100IU;
vitamin E: 5.0 IU to 150 IU, more preferably 120IU;
vitamin C: 30mg to 500mg, more preferably 120mg;
vitamin B1: 0.1mg to 2mg, more preferably 0.25mg;
vitamin B2: 0.1mg to 0.6mg, more preferably 0.3mg;
niacin: 1.5mg to 7.2mg, more preferably 3mg;
vitamin B6: 1.0mg to 3.0mg, more preferably 2mg;
folic acid: 200ug to 600ug, more preferably 400ug;
vitamin B12: 1.5ug to 4.5ug, more preferably 3.8ug;
iron: 2.0mg to 5mg, more preferably 2.75mg; and
zinc: 10mg to 20mg, more preferably 15mg.

Preferably, an embodiment of a composition according to the invention comprises gum acacia.

Preferably, an embodiment of a method according to the invention comprises the steps of blending the nutrients in the required amounts and extruding the blended mixture. More preferably it includes spray drying the mixture.

An advantage of the present invention is that it provides a nutritional composition that can be dissolved instantaneously in water to provide a beverage or soup. It does not require cooking.

Another advantage of the present invention is that it provides a single composition that can be adapted and administered simply in a food for the prevention or treatment of an immune condition in an elderly patient. The composition can be provided in clinical or performance nutrition settings and is particularly suitable for an elderly patient.

Advantages provided by the probiotic bacteria include prevention or inhibition of diarrhea brought about by pathogenic bacteria; prevention or inhibition of diarrhea, especially infections of intestinal cells by rotavirus; prevention of colonization of the intestine by pathogenic bacteria causing diarrhea; and an ability to adhere to and colonise the intestinal mucosa of a host organism.

Yet another advantage of the present invention is that it provides a composition beneficial for diabetics. This is due to the specific composition of macronutrients, protein, carbohydrate and fat which provides a low glycemic index.

Additional features and advantages of the present invention are described in, and will be apparent from the description of the presently preferred embodiments which are set out below.

For the purposes of clarity and a concise description features are described herein as part of the same or separate embodiments, however it will be appreciated that the scope of the invention may include embodiments having combinations of all or some of the features described.

In an embodiment, a nutritional composition comprises a source of protein. The protein source preferably provides 5% to 55% of the energy of the nutritional formula; for example 20% to 50%, preferably 26%, of the energy. Dietary protein is preferred as a source of protein. The dietary protein may be any suitable dietary protein; for example animal protein (such as milk protein, meat protein or egg protein); vegetable protein (such as soy protein, wheat protein, rice protein, and pea protein); a mixture of free amino acids; or a combination thereof. Milk proteins such as casein, whey proteins and soy proteins are particularly preferred.

The composition also comprises a source of carbohydrates and a source of fat.

A fat source preferably provides 5% to 55% of the energy of the nutritional formula; for example 20% to 50%, preferably 23%, of the energy. Lipid making up the fat source may be any suitable fat or fat mixture. Vegetable fat is particularly suitable; for example soy oil, palm oil, coconut oil, safflower oil, sunflower oil, corn oil, canola oil, lecithins, and the like. Most preferably it is a mixture of canola and soy oils. Animal fat such as milk fat may also be added if desired.

Preferably the composition comprises saturated fat which preferably comprises 1% to 5%, more preferably 2.5% of the total energy of the product. Preferably it comprises monounsaturated fat which preferably comprises 5% to 15%, more preferably 9.9% of the total energy of the product. Preferably the composition comprises polyunsaturated fat which preferably comprises 5% to 15%, more preferably 10.1% of the total energy of the product. Preferably the composition comprises linoleic acid which preferably comprises 5% to 15%, more preferably 8.5% of the total energy of the product. Preferably the composition comprises linolenic acid which preferably comprises 0.5% to 5%, more preferably 1.6% of the total energy of the product. Preferably the ratio of linoleic acid to linolenic acid is 3 to 8, more preferably 5.3.

A source of carbohydrate preferably provides 40% to 80%, more preferably 51 % of the energy of the nutritional composition. Any suitable carbohydrate may be used, for example sucrose, lactose, glucose, fructose, corn syrup solids, maltodextrin, or a mixture thereof.

This embodiment of the composition additionally comprises 10E10 cfu of paracaseii or johnsonii probiotic lactic acid bacteria and 6g of fructooligosaccharide and inulin per 300g of the composition.

Dietary fibre may also be added. Preferably dietary fibre provides,up to 5% of the energy of the nutritional composition. The dietary fibre is a mixture of fructooligosaccharide and inulin.

Additional suitable vitamins and minerals are included in the composition as described above.

Monosodium glutamate may be added as a flavour enhancer.

The nutritional composition is preferably enterally administrable; for example in the form of a powder, a liquid concentrate, or a ready-to-drink beverage. If it is desired to produce a powdered nutritional formula, the homogenized mixture is transferred to a suitable drying apparatus such as a spray drier or freeze drier and converted to powder.

Alternatively, a usual food product may be enriched with an embodiment of composition. For example, a fermented milk, a yogurt, a fresh cheese, a renneted milk, an article of confectionery, for example a sweet or sweetened beverage, a confectionery bar, breakfast cereal flakes or bars, a drink, milk powder, soy-based product, non-milk fermented product or a nutritional supplement for clinical nutrition. Then, the amount of the composition added is preferably at least 0.01 % by weight.

The following examples are given by way of illustration only. Percentages and parts are by weight unless otherwise indicated.

### Reference - Example : Nutritional Composition

A nutritional compositions was made by blending the required constituents. The composition is indicated below in table 1. The composition is intended to be consumed in the form of two oral supplements per day based on enriched beverages or milk product desserts. The total daily dose is intended to be approximately 300g or 300ml of composition.

**Table 1**

| | Daily dose 300ml/day |
|---|---|
| Energy P/L/C %TEI | 480kcal (1.6kcal/ml) |
| | 26% / 24% / 51 % |
| Protein g/100 ml | 10.5 (with 6.25 g soy protein) |
| Fat g/100ml | 4.16 |
| Carbohydrate g/100ml | 20.6 |
| in daily dose | per 300ml |
| *Na mg* | 188 |
| *K mg* | 350 |
| *Cl mg* | 290 |
| Ca mg | 200 |
| P mg | 150 |
| *Ca*/*P ratio* | 1.3 |
| Mg mg | 60 |
| Mn ug | 495 |
| A IU | 1333 |
| D IU | 100 |
| E IU | 120 |
| K1 ug | 13.8 |
| C mg | 120 |
| B1 mg | 0.25 |
| B2 mg | 0.3 |
| Niacin mg | 3 |
| B6 mg | 2.0 |
| Folic acid ug | 400 |
| Panto mg | 1.00 |
| B12 ug | 3.8 |
| Biotin ug | 7.5 |
| Fe mg | 2.75 |
| I ug | 75 |
| Cu mg | 1.5 |
| Zn mg | 15 |
| Se ug | 100 |
| Cr ug | 12.5 |
| Mo ug | 18.75 |
| Inulin & FOS blend (30:70 blend) g | 6 |
| Lactobacillus cfu/serving | Paracaseil 10¹⁰ |

## Claims

1. A composition which comprises a source of protein, a source of carbohydrate, a source of fat, a probiotic lactic acid bacterium, fructo-oligosaccharides and inulin, **characterized in that** the probiotic lactic acid bacterium is a paracasei bacteria wherein the lactic acid bacterium is ST11 (deposited under the number CNCM I-2116).

2. A composition according to claim 1 which comprises one or more nutrients or minerals selected from the group consisting of vitamin E, vitamin C, vitamin B6, folic acid, vitamin B 12, copper, zinc and selenium.

3. A composition according to claim 2 wherein the composition comprises at least three nutrients or minerals selected from the group consisting of vitamin E, vitamin C, vitamin B6, folic acid, vitamin B12, copper, zinc and selenium.

4. A composition according to claim 2 or 3 wherein the composition comprises vitamin E, vitamin C, vitamin B6, folic acid, vitamin B12, copper, zinc and selenium

5. A composition according to any preceding claim which comprises one or more nutrients or minerals selected from the group consisting of calcium, phosphorus, magnesium, iron, vitamin A, vitamin B1, vitamin B2, niacin and vitamin D.

6. A composition according to claim 5 wherein the composition comprises at least three nutrients or minerals selected from the group consisting of calcium, phosphorus, magnesium, iron, vitamin A, vitamin B1, vitamin B2, niacin and vitamin D.

7. A composition according to claim 5 or 6 wherein the composition comprises calcium, phosphorus, magnesium, iron, vitamin A, vitamin B1, vitamin B2, niacin and vitamin D.

8. A composition according to any one of claims 5 to 7 wherein calcium is in the form of milk calcium or calcium derived from milk calcium.

9. A composition according to any preceding claim wherein the composition comprises the following masses or amounts of nutrients or minerals (if present) per 300g of the composition:
Vitamin E: 1 IU to 400 IU,
Vitamin C: 6 mg to 300 mg,
Vitamin B6: 0.17 mg to 3 mg,
Folic acid: 40 µg to 800 µg,
Vitamin B12: 0.24 mg to 5mg,
Copper: 0.3 mg to 3 mg,
Zinc: 1.5 mg to 15 mg,
Selenium: 7 µg to 300 µg,
Fructo-oligosaccharides and/or inulin: 4 g to 50 g,
Lactic acid bacteria: 10⁸ to 10¹² cfu.

10. A composition according to any preceding claim wherein the composition comprises the following masses or amounts of nutrients or minerals (if present) per 300g of the composition:
calcium: 100 mg to 300 mg,
phosphorus: 50 mg to 615 mg,
magnesium: 110 mg to 210 mg,
vitamin A: 1000 IU to 1500 IU,
vitamin D: 50 IU to 150 IU,
vitamin E: 5.0 IU to 150 IU,
vitamin C: 30 mg to 500 mg,
vitamin B 1: 0.1 mg to 2 mg,
vitamin B2: 0.1 mg to 0.6 mg,
niacin: 1.5 mg to 7.2 mg,
vitamin B6: 1.0 mg to 3.0 mg,
folic acid: 200 µg to 600 µg,
vitamin B12: 1.5 µg to 4.5 µg,
iron: 2.0 mg to 5 mg,
zinc: 10 mg to 20 mg.

11. A composition according to any preceding claim which comprises gum acacia.

12. A method of producing a composition according to any preceding claim which comprises the step of blending the required nutrients together in the required amounts.

13. A method according to claim 12 which comprises the additional steps of extruding the blended mixture and/or spray drying the mixture.

14. Use of a composition according to any one of claims 1 to 11 in the manufacture of a functional food or a medicament for the prevention or treatment of an immune condition in an elderly patient.

## Patentansprüche

1. Zusammensetzung, die eine Proteinquelle, eine Kohlenhydratquelle, eine Fettquelle, ein probiotisches Milchsäurebakterium, Fructooligosaccharide und Inulin aufweist, **dadurch gekennzeichnet, dass** das probiotische Milchsäurebakterium ein *paracasei-*Bakterium ist, wobei das Milchsäurebakterium ST11 ist (hinterlegt unter der Nummer CNCM 1-2116).

2. Zusammensetzung nach Anspruch 1, die einen oder mehrere Nährstoffe oder Mineralstoffe aufweist, die aus der Gruppe ausgewählt sind, die Vitamin E, Vitamin C, Vitamin B6, Folsäure, Vitamin B 12, Kupfer, Zink und Selen aufweist.

3. Zusammensetzung nach Anspruch 2, wobei die Zusammensetzung mindestens drei Nährstoffe oder Mineralstoffe aufweist, die aus der Gruppe ausgewählt sind, die Vitamin E, Vitamin C, Vitamin B6, Folsäure, Vitamin B 12, Kupfer, Zink und Selen aufweist.

4. Zusammensetzung nach Anspruch 2 oder 3, wobei die Zusammensetzung Vitamin E, Vitamin C, Vitamin B6, Folsäure, Vitamin B 12, Kupfer, Zink und Selen aufweist.

5. Zusammensetzung nach einem der vorhergehenden Ansprüche, die einen oder mehrere Nährstoffe oder Mineralstoffe aufweist, die aus der Gruppe ausgewählt sind, die Kalzium, Phosphor, Magnesium, Eisen, Vitamin A, Vitamin B1, Vitamin B2, Niacin und Vitamin D aufweist.

6. Zusammensetzung nach Anspruch 5, wobei die Zusammensetzung mindestens drei Nährstoffe oder Mineralstoffe aufweist, die aus der Gruppe ausgewählt sind, die Kalzium, Phosphor, Magnesium, Eisen, Vitamin A, Vitamin B1, Vitamin B2, Niacin und Vitamin D aufweist.

7. Zusammensetzung nach Anspruch 5 oder 6, wobei die Zusammensetzung Kalzium, Phosphor, Magnesium, Eisen, Vitamin A, Vitamin B1, Vitamin B2, Niacin und Vitamin D aufweist.

8. Zusammensetzung nach einem der Ansprüche 5 bis 7, wobei Kalzium die Form von Milchkalzium oder aus Milchkalzium abgeleitetem Kalzium hat.

9. Zusammensetzung nach einem der vorhergehenden Ansprüche, wobei die Zusammensetzung die folgenden Nährstoff- oder Mineralstoffmengen (sofern vorhanden) je 300g der Zusammensetzung aufweist:
Vitamin E: 1 IE bis 400 IE,
Vitamin C: 6 mg bis 300 mg,
Vitamin B6: 0,17 mg bis 3 mg,
Folsäure: 40 µg bis 800 µg,
Vitamin B12: 0,24 mg bis 5mg,
Kupfer: 0,3 mg bis 3 mg,
Zink: 1,5 mg bis 15 mg,
Selen: 7 µg bis 300 µg,
Fructooligosaccharide und/oder Inulin: 4 g bis 50 g,
Milchsäurebakterien: 10⁸ bis 10¹² KbE.

10. Zusammensetzung nach einem der vorhergehenden Ansprüche, wobei die Zusammensetzung die folgenden Nährstoff- oder Mineralstoffmengen (sofern vorhanden) je 300g der Zusammensetzung aufweist:
Kalzium: 100 mg bis 300 mg,
Phosphor: 50 mg bis 615 mg,
Magnesium: 110 mg bis 210 mg,
Vitamin A: 1000 IE bis 1500 IE,
Vitamin D: 50 IE bis 150 IE,
Vitamin E: 5,0 IE bis 150 IE,
Vitamin C: 30 mg bis 500 mg,
Vitamin B1: 0,1 mg bis 2 mg,
Vitamin B2: 0,1 mg bis 0,6 mg,
Niacin: 1,5 mg bis 7,2 mg,
Vitamin B6: 1,0 mg bis 3,0 mg,
Folsäure 200 µg bis 600 µg,
Vitamin B12:1,5 mg bis 4,5 µg,
Eisen: 2,0 mg bis 5 mg,
Zink: 10 mg bis 20 mg.

11. Zusammensetzung nach einem der vorhergehenden Ansprüche, die Gummi arabicum aufweist.

12. Verfahren zur Herstellung einer Zusammensetzung nach einem der vorhergehenden Ansprüche, die den Schritt des Mischens der erforderlichen Nährstoffe in den erforderlichen Mengen aufweist.

13. Verfahren nach Anspruch 12, das die weiteren Schritte des Extrudierens der Mischung und/oder des Sprühtrocknens der Mischung aufweist.

14. Verwendung einer Zusammensetzung nach einem der Ansprüche 1 bis 11 in der Herstellung eines funktionellen Lebensmittels oder eines Medikamentes zur Vorbeugung oder Behandlung einer Immunstörung bei älteren Patienten.

## Revendications

1. Composition qui comprend une source de protéines, une source de glucides, une source de matière grasse, une bactérie lactique probiotique, des fructo-oligosaccharides et de l'inuline,
**caractérisée en ce que** la bactérie lactique probiotique est une bactérie paracasei, dans laquelle la bactérie lactique est ST11 (déposée sous le numéro CNCM I-2116).

2. Composition selon la revendication 1, qui comprend un ou plusieurs nutriments ou minéraux choisis parmi le groupe constitué de vitamine E, vitamine C, vitamine B6, acide folique, vitamine B 12, cuivre, zinc et sélénium.

3. Composition selon la revendication 2, la composition comprenant au moins trois nutriments ou minéraux choisis parmi le groupe constitué de vitamine E, vitamine C, vitamine B6, acide folique, vitamine B12, cuivre, zinc et sélénium.

4. Composition selon la revendication 2 ou 3, la composition comprenant vitamine E, vitamine C, vitamine B6, acide folique, vitamine B12, cuivre, zinc et sélénium.

5. Composition selon l'une quelconque des revendications précédentes, qui comprend un ou plusieurs nutriments ou minéraux choisis parmi le groupe constitué de calcium, phosphore, magnésium, fer, vitamine A, vitamine B1, vitamine B2, niacine et vitamine D.

6. Composition selon la revendication 5, la composition comprenant au moins trois nutriments ou minéraux choisis parmi le groupe constitué de calcium, phosphore, magnésium, fer, vitamine A, vitamine B1, vitamine B2, niacine et vitamine D.

7. Composition selon la revendication 5 ou 6, la composition comprenant calcium, phosphore, magnésium, fer, vitamine A, vitamine B1, vitamine B2, niacine et vitamine D.

8. Composition selon l'une quelconque des revendications 5 à 7, dans laquelle le calcium est sous la forme de calcium de lait ou de calcium dérivé de calcium de lait.

9. Composition selon l'une quelconque des revendications précédentes, la composition comprenant les masses ou quantités suivantes de nutriments ou minéraux (si ils sont présents) pour 300 g de la composition :
Vitamine E : 1 UI à 400 UI,
Vitamine C : 6 mg à 300 mg,
Vitamine B6 : 0,17 mg à 3 mg,
Acide folique : 40 µg à 800 µg,
Vitamine B12 : 0,24 mg à 5 mg,
Cuivre : 0,3 mg à 3 mg,
Zinc : 1,5 mg à 15 mg,
Sélénium : 7 µg à 300 µg,
Fructo-oligosaccharides et/ou inuline : 4 g à 50 g,
Bactéries lactiques : 10⁸ à 10¹² ufc.

10. Composition selon l'une quelconque des revendications précédentes, la composition comprenant les masses ou quantités suivantes de nutriments ou minéraux (si ils sont présents) pour 300 g de la composition :
calcium : 100 mg à 300 mg,
phosphore : 50 mg à 615 mg,
magnésium : 110 mg à 210 mg,
vitamine A : 1000 UI à 1500 UI,
vitamine D : 50 UI à 150 UI,
vitamine E : 5,0 UI à 150 UI,
vitamine C : 30 mg à 500 mg,
vitamine B1 : 0,1 mg à 2 mg,
vitamine B2 : 0,1 mg à 0,6 mg,
niacine : 1,5 mg à 7,2 mg,
vitamine B6 : 1,0 mg à 3,0 mg,
acide folique : 200 µg à 600 µg,
vitamine B12 : 1,5 µg à 4,5 µg,
fer : 2,0 mg à 5 mg,
zinc : 10 mg à 20 mg.

11. Composition selon l'une quelconque des revendications précédentes, qui comprend de la gomme d'acacia.

12. Procédé de production d'une composition selon l'une quelconque des revendications précédentes, qui comprend l'étape consistant à mélanger ensemble les nutriments requis dans les quantités requises.

13. Procédé selon la revendication 12, qui comprend les étapes supplémentaires consistant à extruder la mixture mélangée et/ou sécher par pulvérisation la mixture.

14. Utilisation d'une composition selon l'une quelconque des revendications 1 à 11 dans la fabrication d'un aliment fonctionnel ou d'un médicament pour la prévention ou le traitement d'un état immunitaire chez un patient âgé.
